# EUROPEAN PATENT APPLICATION

(11) **EP 4 740 893 A2**
(43) Date of publication of application: **13.05.2026**
(21) Application number: 26168281.9
(22) Date of filing: 15.06.2021
(51) Int. Cl.: A61B 90/00

(54) **USER INPUT SYSTEMS FOR A COMPUTER-ASSISTED MEDICAL SYSTEM**

(30) Priority: 16.06.2020 US 202063039753 P
(62) Divisional of application: 21740320.3
(71) Applicant: Intuitive Surgical Operations, Inc., Sunnyvale, CA 94086 (US)
(72) Inventor: SHADEMAN, Azad, Sunnyvale, CA 94086 (US); AZIZIAN, Mahdi, Sunnyvale, CA 94086 (US); PROKSCH, Daniel, Sunnyvale, CA 94086 (US); SHIRAZIAN, Pourya, Sunnyvale, CA 94086 (US)
(74) Representative: Mathys & Squire

(57) **Abstract**

A user input system directs a computer-assisted medical system, which includes a set of controls comprising a first control and a second control configured to receive user input, to operate in a manipulation input mode in which user input to the first control manipulates a first manipulator of the computer-assisted medical system, and user input to the second control manipulates a second manipulator of the computer-assisted medical system. The user input system further directs the computer-assisted medical system to operate in a hybrid input mode in which user input to the first control manipulates the first manipulator, and user input to the second control adjusts a parameter setting associated with a medical procedure.

## Description

### RELATED APPLICATIONS

The present application claims priority to U.S. Provisional Patent Application No. 63/039,753, filed June 16, 2020, the contents of which are hereby incorporated by reference in their entirety.

### BACKGROUND INFORMATION

Various technologies including computing technologies, robotic technologies, medical technologies, and extended reality technologies (e.g., augmented reality technologies, virtual reality technologies, etc.) have made it possible for users such as surgeons to perform, and be trained to perform, various types of medical procedures. For example, users may perform and be trained to perform minimally-invasive medical procedures such as computer-assisted medical procedures in clinical settings (e.g., procedures on bodies of live human or animal patients), in non-clinical settings (e.g., procedures on bodies of human or animal cadavers, bodies of tissue removed from human or animal anatomies, etc.), in training settings (e.g., procedures on bodies of physical anatomical training models, bodies of virtual anatomy models in extended reality environments, etc.), and so forth.

During a procedure in any such setting, a user may view imagery of an anatomical space associated with a body (e.g., an area internal to the body) as the user controls instruments of a computer-assisted medical system to perform the procedure. The user may control the instruments using various input mechanisms on or coupled to the computer-assisted medical system. The input mechanisms may affect how efficiently and/or effectively the user is able to perform a procedure.

### SUMMARY

The following description presents a simplified summary of one or more aspects of the systems and methods described herein. This summary is not an extensive overview of all contemplated aspects and is intended to neither identify key or critical elements of all aspects nor delineate the scope of any or all aspects. Its sole purpose is to present one or more aspects of the systems and methods described herein as a prelude to the detailed description that is presented below.

An illustrative system includes a memory storing instructions and a processor communicatively coupled to the memory and configured to execute the instructions to direct a computer-assisted medical system, which includes a set of controls comprising a first control and a second control configured to receive user input, to operate in a manipulation input mode in which user input to the first control manipulates a first manipulator of the computer-assisted medical system, and user input to the second control manipulates a second manipulator of the computer-assisted medical system; and direct the computer-assisted medical system to operate in a hybrid input mode in which user input to the first control manipulates the first manipulator, and user input to the second control adjusts a parameter setting associated with a medical procedure.

An illustrative method includes a processor (e.g., a processor of a user input system) directing a computer-assisted medical system, which includes a set of controls comprising a first control and a second control configured to receive user input, to operate in a manipulation input mode in which user input to the first control manipulates a first manipulator of the computer-assisted medical system, and user input to the second control manipulates a second manipulator of the computer-assisted medical system; and directing the computer-assisted medical system to operate in a hybrid input mode in which user input to the first control manipulates the first manipulator, and user input to the second control adjusts a parameter setting associated with a medical procedure.

An illustrative computer-readable medium includes instructions that, when executed by a processor, cause the processor to direct a computer-assisted medical system, which includes a set of controls comprising a first control and a second control configured to receive user input, to operate in a manipulation input mode in which user input to the first control manipulates a first manipulator of the computer-assisted medical system, and user input to the second control manipulates a second manipulator of the computer-assisted medical system; and direct the computer-assisted medical system to operate in a hybrid input mode in which user input to the first control manipulates the first manipulator, and user input to the second control adjusts a parameter setting associated with a medical procedure.

An illustrative system includes a memory storing instructions and a processor communicatively coupled to the memory and configured to execute the instructions to direct a computer-assisted medical system, which includes a set of controls comprising a first control and a second control configured to receive user input, to operate in a manipulation input mode in which the first control is configured to receive a first input to manipulate a first manipulator of the computer-assisted medical system, and the second control is configured to receive a second input to manipulate a second manipulator of the computer-assisted medical system; and direct the computer-assisted medical system to operate in a hybrid input mode in which the first control is configured to receive a third input to manipulate the first manipulator, and the second control is configured to receive a fourth input to adjust a parameter setting associated with a medical procedure.

An illustrative method includes a processor (e.g., a processor of a user input system) directing a computer-assisted medical system, which includes a set of controls comprising a first control and a second control configured to receive user input, to operate in a manipulation input mode in which the first control is configured to receive a first input to manipulate a first manipulator of the computer-assisted medical system, and the second control is configured to receive a second input to manipulate a second manipulator of the computer-assisted medical system; and directing the computer-assisted medical system to operate in a hybrid input mode in which the first control is configured to receive a third input to manipulate the first manipulator, and the second control is configured to receive a fourth input to adjust a parameter setting associated with a medical procedure.

An illustrative computer-readable medium includes instructions that, when executed by a processor, cause the processor to direct a computer-assisted medical system, which includes a set of controls comprising a first control and a second control configured to receive user input, to operate in a manipulation input mode in which the first control is configured to receive a first input to manipulate a first manipulator of the computer-assisted medical system, and the second control is configured to receive a second input to manipulate a second manipulator of the computer-assisted medical system; and direct the computer-assisted medical system to operate in a hybrid input mode in which the first control is configured to receive a third input to manipulate the first manipulator, and the second control is configured to receive a fourth input to adjust a parameter setting associated with a medical procedure.

### BRIEF DESCRIPTION OF THE DRAWINGS

The accompanying drawings illustrate various embodiments and are a part of the specification. The illustrated embodiments are merely examples and do not limit the scope of the disclosure. Throughout the drawings, identical or similar reference numbers designate identical or similar elements.
FIG. 1 illustrates an example user input system according to principles described herein.
FIGS. 2A and 2B illustrate an example configuration of a user input system for a computer-assisted medical system according to principles described herein.
FIG. 3 illustrates an example image from a computer-assisted medical system according to principles described herein.
FIGS. 4A-5B illustrate example interfaces for a user input system for a computer-assisted medical system according to principles described herein.
FIG. 6 illustrates an example user input method for a computer-assisted medical system according to principles described herein.
FIG. 7 illustrates an example user input method for a computer-assisted medical system according to principles described herein.
FIG. 8 illustrates an example user input method for a computer-assisted medical system according to principles described herein.
FIG. 9 illustrates an example computer-assisted medical system according to principles described herein.
FIG. 10 illustrates an example computing device according to principles described herein.

### DETAILED DESCRIPTION

User input systems and methods for a computer-assisted medical system are described herein. During a computer-assisted medical procedure, a user (e.g., a surgeon) may control (e.g., teleoperate) instruments through a set of controls that receive inputs configured to cause the instruments to be manipulated by the computer-assisted medical system. For example, user inputs received through the set of controls may cause elements of the computer-assisted medical system, such as manipulators of the computer-assisted medical system, to be manipulated or otherwise controlled in a manner that manipulates instruments connected to the manipulators.

The set of controls may be configured to operate in a manipulation input mode in which user inputs to the controls (e.g., manipulation of the controls by a user) may translate to manipulation of the instruments and/or manipulators by the computer-assisted medical system. As described herein, the set of controls may be further configured to operate in a hybrid input mode in which user input to one of the controls causes the computer-assisted medical system to manipulate an instrument and/or a manipulator and user input to another one of the controls causes one or more parameter settings associated with the computer-assisted medical procedure to be adjusted. When operating in the hybrid input mode, for example, user inputs to one control may be translated to manipulation (e.g., movement) of the instrument and/or manipulator while user inputs to another control may be translated to adjustment of a parameter setting associated with the medical procedure. Examples of such a parameter setting are described herein.

Systems and methods described herein may provide various advantages and benefits. For example, systems and methods described herein provide a hybrid input mode in which a set of user controls are configured to receive inputs for manipulating an instrument while adjusting a parameter setting associated with a medical procedure, such as a parameter setting of the instrument. The hybrid input mode may increase efficiency and/or effectiveness of receiving inputs to a computer-assisted medical system when compared to conventional user input systems for computer-assisted medical systems (e.g., conventional user input systems in which manipulation of instruments and adjustment of parameter settings must be performed at different times and/or in separate input modes, conventional user input systems in which separate input mechanisms other than a set of controls used to manipulate instruments must be used to adjust parameters settings, etc.). Such increase in efficiency and/or effectiveness of receiving inputs may result in more efficient and/or effective medical procedures, such as by facilitating more efficient and/or effective operation of a medical instrument. These and other advantages and benefits of systems and methods described herein will be made apparent herein.

Various embodiments will now be described in more detail with reference to the figures. The disclosed systems and methods may provide one or more of the benefits mentioned above and/or various additional and/or alternative benefits that will be made apparent herein.

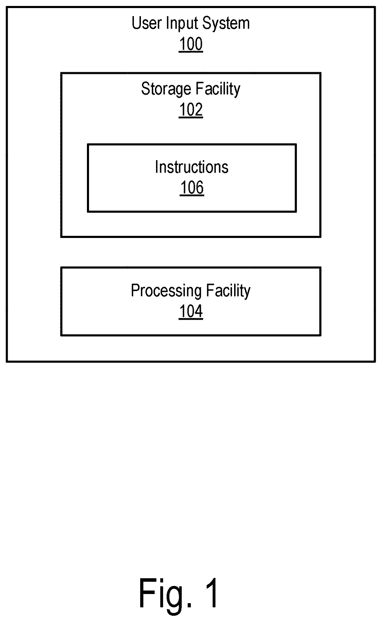

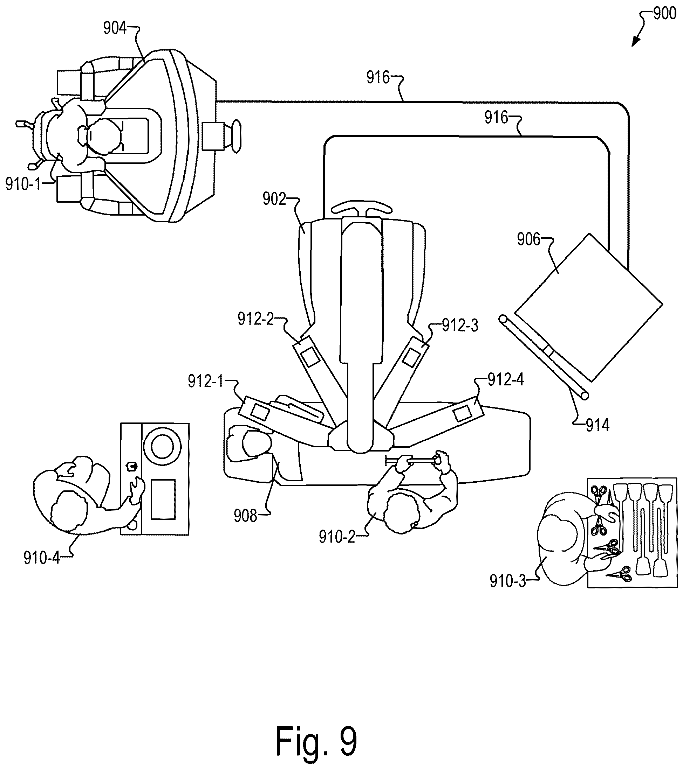

FIG. 1 illustrates an example user input system 100 ("system 100") for a computer-assisted medical system. System 100 may be included in, implemented by, or connected to one or more components of a computer-assisted medical system such as an illustrative computer-assisted medical system that will be described below in relation to FIG. 9. For example, system 100 may be implemented by one or more components of a computer-assisted medical system such as a manipulating system, a user control system, or an auxiliary system. As another example, system 100 may be implemented by a stand-alone computing system communicatively coupled to a computer-assisted medical system.

As shown in FIG. 1, system 100 may include, without limitation, a storage facility 102 and a processing facility 104 selectively and communicatively coupled to one another. Facilities 102 and 104 may each include or be implemented by one or more physical computing devices including hardware and/or software components such as processors, memories, storage drives, communication interfaces, instructions stored in memory for execution by the processors, and so forth. Although facilities 102 and 104 are shown to be separate facilities in FIG. 1, facilities 102 and 104 may be combined into fewer facilities, such as into a single facility, or divided into more facilities as may serve a particular implementation. In some examples, each of facilities 102 and 104 may be distributed between multiple devices and/or multiple locations as may serve a particular implementation.

Storage facility 102 may maintain (e.g., store) executable data used by processing facility 104 to perform any of the functionality described herein. For example, storage facility 102 may store instructions 106 that may be executed by processing facility 104 to perform one or more of the operations described herein. Instructions 106 may be implemented by any suitable application, software, code, and/or other executable data instance. Storage facility 102 may also maintain any data received, generated, managed, used, and/or transmitted by processing facility 104.

Processing facility 104 may be configured to perform (e.g., execute instructions 106 stored in storage facility 102 to perform) various operations associated with a user input system for a computer-assisted medical system. Examples of such operations that may be performed by system 100 (e.g., by processing facility 104 of system 100) are described herein. In the description that follows, any references to functions performed by system 100 may be understood to be performed by processing facility 104 based on instructions 106 stored in storage facility 102.

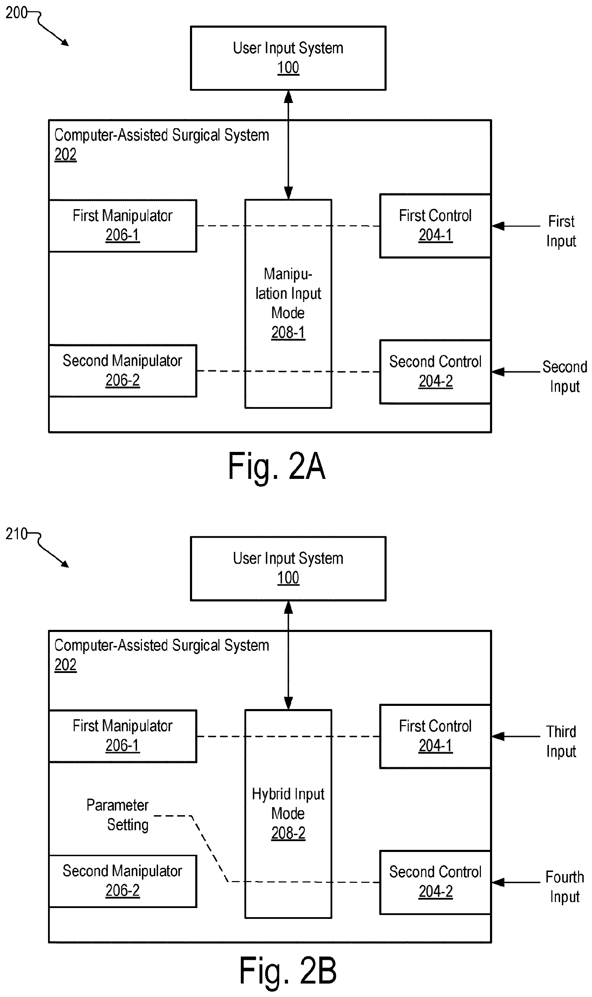

FIG. 2A illustrates an example configuration 200 of user input system 100 for a computer-assisted medical system 202. While FIG. 2A shows user input system 100 separate from computer-assisted medical system 202, in some examples user input system 100 may be a component of computer-assisted medical system 202. As shown, computer-assisted medical system 202 includes a set of controls 204 that includes a first control 204-1 and a second control 204-2. Computer-assisted medical system 202 further includes manipulators 206 (e.g., a first manipulator 206-1 and a second manipulator 206-2).

Controls 204 may be implemented in any suitable manner to receive user input for manipulating manipulators 206. For instance, controls 204 may be implemented by a set of master controls, an example of which is described in relation to FIG. 9. Additionally or alternatively, controls 204 may include any other hand-driven controls that may be moved, rotated, pinched and un-pinched by hands that engage the hand-driven controls (e.g., joysticks, directional pads, or other handheld controls), foot-driven controls (e.g., pedals or other controls configured to be manipulated by a foot or feet), head-driven controls (e.g., a helmet or sensors configured to detect movement of a head to manipulate manipulators 206), gaze-based controls (e.g., controls configured to detect eye movement and/or gaze to manipulate manipulators 206), audio-based controls (e.g., controls configured to detect voice commands to manipulate manipulators 206), and/or any other controls configured to receive user input to manipulate manipulators 206.

Manipulators 206 may be implemented in any suitable manner to manipulate and/or control medical instruments based on user input received by controls 204. Medical instruments may include any device, tool, or other instrument that may be used in a medical procedure, such as surgical instruments, non-surgical instruments, imaging devices, etc. Manipulators 206 may include any suitable mechanism that translates input from controls 204 to operation of medical instruments coupled to the manipulators 206. For example, manipulators 206 may include configurations of manipulator arms, motorized joints, and/or other manipulator components that are configured to be moved based on user input received by controls 204. Medical instruments may be coupled to manipulator arms such that movement of the manipulator arms causes movement of the medical instruments.

Configuration 200 illustrates user input system 100 configuring computer-assisted medical system 202 to operate in a manipulation input mode 208-1. In manipulation input mode 208-1, computer-assisted medical system 202 may be configured to translate user input received by way of controls 204 to manipulation (e.g., movement) of manipulators 206 and/or instruments connected to manipulators 206. For instance, computer-assisted medical system 202 may receive a first input on first control 204-1 and a second input on second control 204-2. User input system 100 may translate the first input to a manipulation of first manipulator 206-1 and the second input to a manipulation of second manipulator 206-2. As an example, the first input may be a movement of first control 204-1 from right to left. In response, user input system 100 may translate the movement of first control 204-1 to a corresponding movement of an instrument coupled to first manipulator 206-1 (e.g., a movement of the instrument a corresponding amount from right to left based on the input movement). The second input may be a counterclockwise twisting of second control 204-2. In response, user input system 100 may translate the twisting of second control 204-2 to a corresponding rotation of another instrument coupled to second manipulator 206-2 (e.g., a counterclockwise rotation by an amount corresponding to the counterclockwise twisting input). Any other suitable manipulations (e.g., any changes in pose such as changes in position and/or orientation) of controls 204 through user inputs (e.g., movements of hands, wrists, arms, etc., a movement of fingers attached to finger portions of controls 204, an actuation of a button or other input mechanism to send a control signal, etc.) may be considered input received by controls 204 and may be translated into corresponding manipulations of manipulators 206 and/or instruments coupled to manipulators 206 by user input system 100 operating in manipulation input mode 208-1.

As used herein, manipulation of a control, a manipulator, and/or an instrument generally refers to physical movement of the control, the manipulator, and/or the instrument. In certain implementations, for example, user input may be provided to a control that causes the control to be moved within a space. User input system 100 may translate the user input to manipulation of a manipulator and/or an instrument coupled to the manipulator.

FIG. 2B illustrates another example configuration 210 of user input system 100 for computer-assisted medical system 202. In configuration 210, user input system 100 configures computer-assisted medical system 202 to operate in a hybrid input mode 208-2. In hybrid input mode 208-2, computer-assisted medical system 202 may be configured to translate input received on one of controls 204 into manipulation of one of manipulators 206, while input received on another of controls 204 may adjust a parameter setting associated with a medical procedure. For instance, computer-assisted medical system 202 may receive a third input on first control 204-1 and a fourth input on second control 204-2. User input system 100 may translate the third input to a manipulation of first manipulator 206-1, as in manipulation input mode 208-1. However, rather than translate the fourth input to a manipulation of second manipulator 206-2, user input system 100 may adjust a parameter setting based on the fourth input received on second control 204-2. In some instances, the third input and the fourth input may be received concurrently (e.g., simultaneously, within a substantially short time period, etc.) by user input system 100. Thus, user input system 100 may enable a user to manipulate a medical instrument (via the third input on first control 204-1) while also adjusting one or more parameter settings associated with the medical procedure (via the fourth input on second control 204-2). As an example, the user may move a cauterizing instrument coupled to first manipulator 206-1 using first control 204-1 while adjusting a power output of the cauterizing instrument using second control 204-2.

Parameter settings associated with the medical procedure may include any adjustable properties and/or characteristics of the computer-assisted medical system, adjustable properties and/or characteristics of any of the medical instruments involved with the medical procedure, adjustable properties and/or characteristics of any data provided to the computer-assisted medical system or by the computer-assisted medical system (e.g., display properties, user interface properties, etc.). For example, a parameter setting associated with the medical procedure may include a display parameter setting of a display device associated with the medical procedure (e.g., a brightness, color, saturation, hue, image layout, image format, etc. associated with the display device and/or content displayed by the display device such as a three-dimensional (3D) model or any other content), an imaging parameter setting of an imaging device and/or an image processing process associated with the medical procedure (e.g., one or more auto-exposure settings of an imaging device), an operation parameter setting of a medical instrument associated with the medical procedure, a user interface parameter setting of a user interface associated with the computer-assisted medical system, and/or any other adjustable parameter setting of the computer-assisted medical system and/or a medical instrument used in or otherwise associated with the medical procedure.

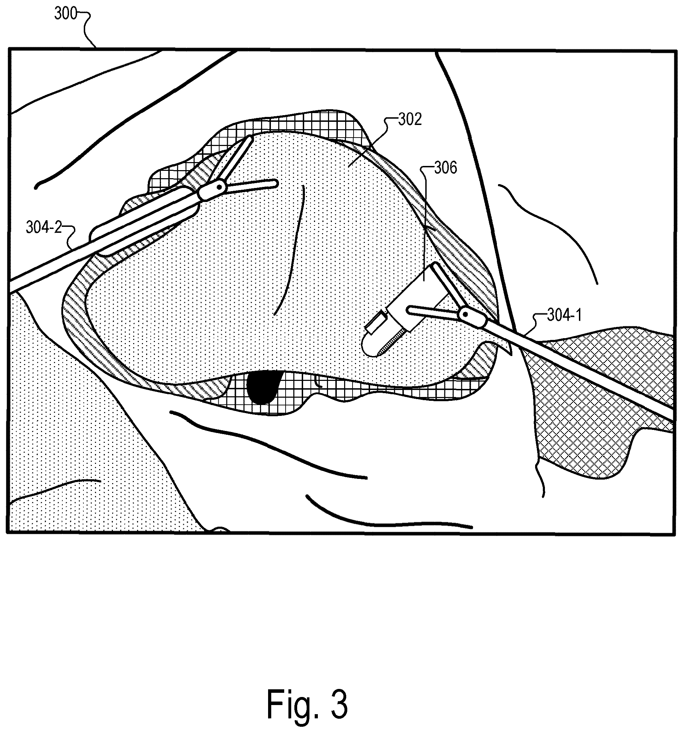

To illustrate, a user may operate a computer-assisted medical system to control a medical instrument such as an ultrasound probe located at an anatomical space associated with a medical procedure. Imagery of the anatomical space may be captured and presented in conjunction with the medical procedure. FIG. 3 shows an illustrative image 300 provided by the computer-assisted medical system. Image 300 may be an image of the anatomical space provided by an imaging device (e.g., an imaging device included in or coupled to the computer-assisted medical system). Image 300 shows an anatomical object 302, which may be an organ on which the user is performing the medical procedure. Image 300 also shows a first instrument 304-1 and a second instrument 304-2. First instrument 304-1 may be coupled to a first manipulator of the computer-assisted medical system and second instrument 304-2 may be coupled to a second manipulator of the computer-assisted medical system. First instrument 304-1 is further coupled to an ultrasound probe 306 (e.g., by grasping or otherwise engaging a drop-in ultrasound probe). The user may control ultrasound probe 306 through the computer-assisted medical system.

When system 100 configures the computer-assisted medical system to operate in a manipulation input mode, first instrument 304-1 and second instrument 304-2 may be controlled by the first and second manipulators, which are in turn controlled by a user providing input via a set of controls of the computer-assisted medical system. For example, the user may provide input to a first control of the set of controls to manipulate the first manipulator to cause first instrument 304-1 to move (e.g., by changing a position and/or orientation of) ultrasound probe 306 in the anatomical space (e.g., so as to capture ultrasound imagery at different locations in the anatomical space, such as at different locations on anatomical object 302). The user may further provide input to a second control of the set of controls to manipulate the second manipulator to cause second instrument 304-2 to move in the anatomical space (e.g., to perform any suitable tasks in the anatomical space).

When system 100 configures the computer-assisted medical system to operate in a hybrid input mode, the user may manipulate first instrument 304-1 by providing input to the first control to manipulate the first manipulator. In this way, the user may continue to move a position and/or orientation of ultrasound probe 306 after system 100 switches from the manipulation input mode to the hybrid input mode during the medical procedure. In the hybrid input mode, the user may also adjust one or more parameter settings associated with the medical procedure by providing input to the second control. To this end, any of user input that may be provided to the second control for manipulating the second manipulator during operation in the manipulation input mode may instead be translated, by system 100, to commands for adjusting parameter settings during operation in the hybrid input mode. For example, in the manipulation input mode, a clockwise twisting of the second control may result in a clockwise movement of the second manipulator and accordingly, second instrument 304-2. In hybrid input mode, the clockwise twisting of the second control may instead be translated to an adjustment to a parameter setting associated with the medical procedure, such as an increasing of a value of a parameter setting associated with the medical procedure.

In hybrid input mode, system 100 may be configured to translate any user input received by the second control into an adjustment of a parameter setting in any suitable way. As an example, specific user inputs received by the second control may be translated into specific corresponding adjustments of a parameter setting (e.g., a clockwise twisting input is translated to an increase in a value of a parameter setting, a counter-clockwise twisting input is translated to a decrease in a value of a parameter setting, a pinching input is translated to an adjustment of a parameter setting, etc.). As another example, specific user inputs received by the second control may be translated into operations in a user interface (e.g., a graphical user interface), which operations may be performed to adjust a parameter setting. For instance, user input received by the second control may cause a cursor to be moved and/or otherwise used within a graphical user interface to adjust a parameter setting. Examples of user interfaces that may be provided by system 100 and used to facilitate adjustment of parameter settings in hybrid input mode will now be described.

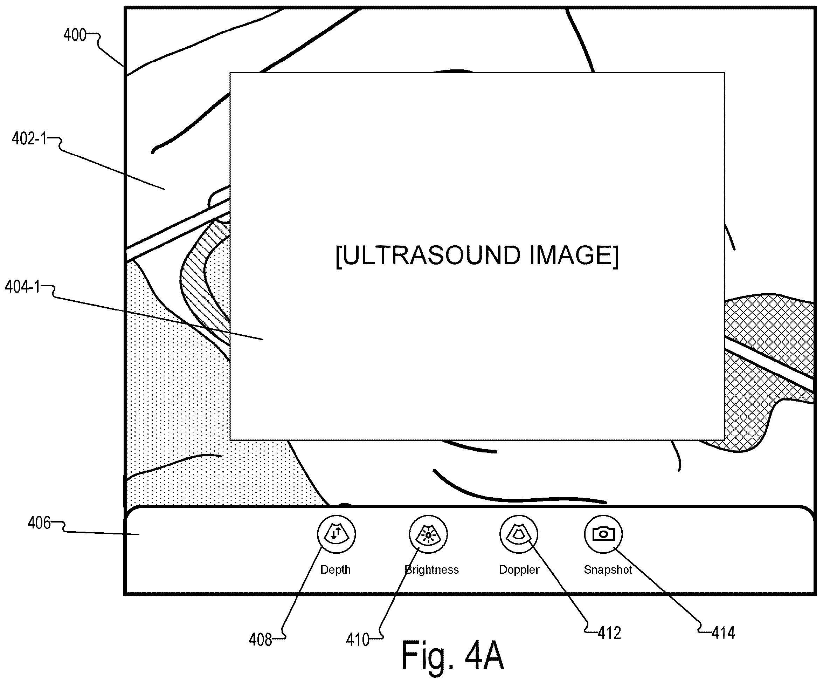

FIGS. 4A-4E illustrate an example interface 400 for a hybrid input mode for a computer-assisted medical system. Interface 400 includes images 402 (e.g., image 402-1 through 402-5) of the anatomical space (e.g., endoscopic images of the anatomical scene) and ultrasound images 404 (ultrasound image 404-1 through 404-5), which may be captured by ultrasound probe 306 manipulated by first instrument 304-1 coupled to the first manipulator of the computer-assisted medical system. In this example, images 402 may be similar to image 300 (e.g., depicting the anatomical space shown in image 300 at different points in time). An ultrasound image 404 may be displayed together with an image 402 in interface 400 in any suitable configuration. In some configurations, the ultrasound image 404 may overlap and/or partially obstruct elements of image 402 from view. While user interface 400 shows ultrasound images 404 positioned centrally in view and obstructing a significant portion of images 402, the illustrated example is illustrative only and not limiting. An ultrasound image 404 may be positioned, within a user interface, at any suitable place relative to an image 402, including at positions configured to reduce, minimize, or eliminate any overlap of image 402 by ultrasound image 404. For example, ultrasound image 404 may be smaller than shown in user interface 400 and may be positioned in a corner area of user interface 400 to minimize an extent of obstruction or avoid obstructing an area and/or elements of interest (e.g., medical instruments) depicted in image 402. For instance, ultrasound image 404 may be configured to allow ultrasound probe 306 to be unobstructed and remain in view to the user, which may allow the user to concurrently use the first control to move ultrasound probe 306 in the anatomical space and the second control to adjust one or more parameter settings associated with ultrasound probe 306.

Interface 400 further includes a user interface element 406 that includes icons 408-414 that indicate parameter settings that may be adjusted by the user via the second control in the hybrid input mode. In this example, icons 408-414 include a depth icon 408, a brightness icon 410, a doppler icon 412, and a snapshot icon 414. Depth icon 408 may allow the user to adjust a depth of focus of ultrasound images 404 captured by ultrasound probe 306. Brightness icon 410 may allow the user to adjust a brightness of the display of ultrasound images 404. Doppler icon 412 may allow the user to change a mode of the ultrasound (e.g., turning on or off a doppler mode). Snapshot icon 414 may allow the user to store still images or portions of imagery from ultrasound images 404 received from ultrasound probe 306. Thus, while the user is adjusting any of these (or any other suitable) parameter settings using the second control, the user may also move ultrasound probe 306 using the first control to capture ultrasound imagery from different locations in the anatomical space, such as different parts and/or angles of anatomical object 302.

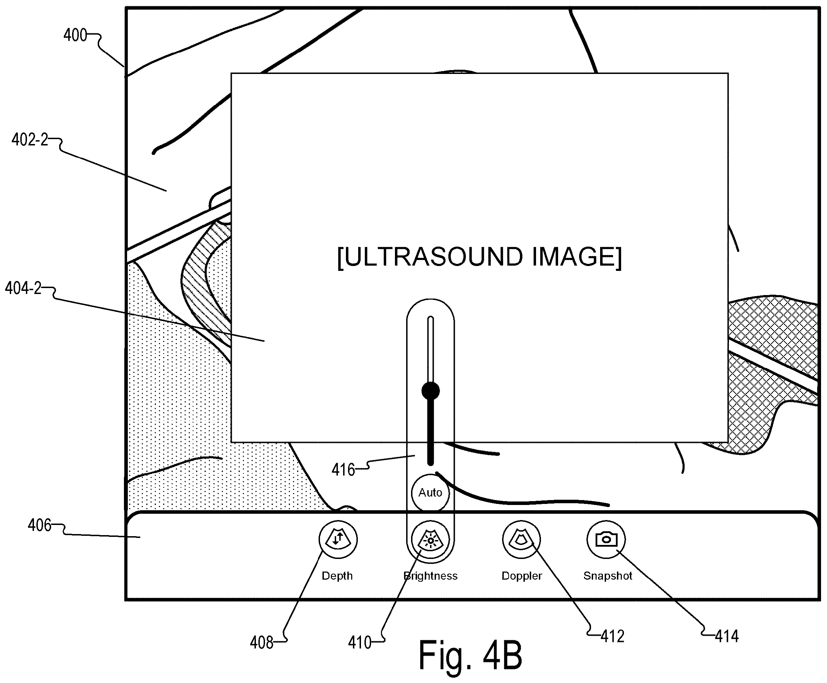

For example, FIG. 4B illustrates the user adjusting a brightness of an ultrasound image 404-2 in interface 400 in the hybrid input mode. The user may adjust the brightness by using the second control to interact with brightness icon 410. The hybrid input mode may allow the user to select and/or interact with brightness icon 410 in any suitable manner. For instance, the hybrid input mode may allow the user to control a cursor on interface 400 via the second control, which the user may use to indicate and make selections on interface 400. The hybrid input mode may allow the user to move the cursor by moving the second control and make selections via a button or a finger input (e.g., a pinch gesture) or any other suitable input mechanism on the second control. Additionally or alternatively, the hybrid mode may allow the user to cycle through a predetermined set of options (e.g., icons 408-414 or any other parameter settings provided for adjustment) and make selections to adjust parameter settings. For example, the hybrid input mode may allow the user to pick a next option by moving the second control a certain direction and a previous option by moving the second control an opposite direction, or any other suitable input mechanism on the second control. The user may cycle through options, such as icons 408-414, through such input mechanisms and make a selection of an icon to adjust parameter settings associated with the selected icon.

As shown in FIG. 4B, the user has selected brightness icon 410. Based on the selection, user interface element 406 may display a brightness slider 416 to allow the user to adjust the brightness of ultrasound image 404-2. The user may interact with brightness slider 416 in a manner similar to the user's selection of brightness icon 410. For example, the hybrid input mode may allow the user to control a cursor and select a brightness setting on brightness slider 416 using the second control. Additionally or alternatively, upon selecting brightness icon 410, the user may raise and lower brightness slider 416 using the second control by moving the second control up and down, twisting the second control clockwise and counterclockwise, pushing buttons on the second control and/or any other suitable input mechanism of the second control. Based on an adjustment of the brightness parameter setting via slider 416, a brightness of ultrasound image 404-2 may be adjusted.

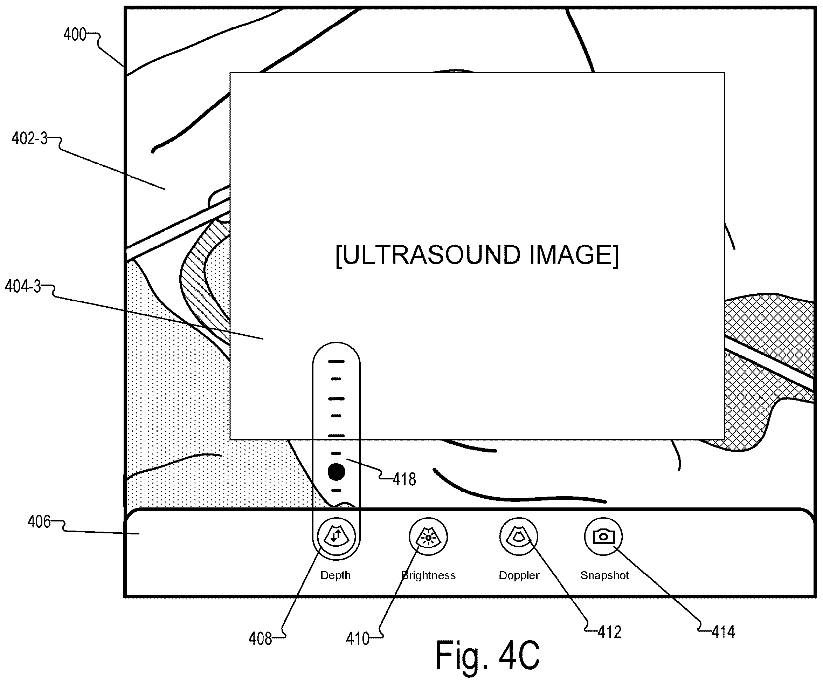

FIG. 4C illustrates an example in which the user adjusts a depth of ultrasound image capture in the hybrid input mode. As the user adjusts the depth of ultrasound scan in interface 400, one or more depth scanning parameters of the ultrasound probe may be adjusted to change the ultrasound imagery displayed in ultrasound image 404-3. The adjustments and changes may occur in real time.

The interaction with interface 400 shown in FIG. 4C may be similar to the interaction described in relation to FIG. 4B. As with brightness icon 410, the computer-assisted medical system may allow the user to select depth icon 408 using the second control in the hybrid input mode. Based on the selection of depth icon 408, user interface element 406 may display a depth slider 418 that allows the user to select a depth of focus of ultrasound probe 306. Based on the depth selection, ultrasound image 404-3 may be an ultrasound image from deeper or shallower within the tissue of a target of ultrasound probe 306, such as anatomical object 302. The hybrid input mode may allow the user to interact with depth icon 408 and depth slider 418 in any suitable manner, as described herein.

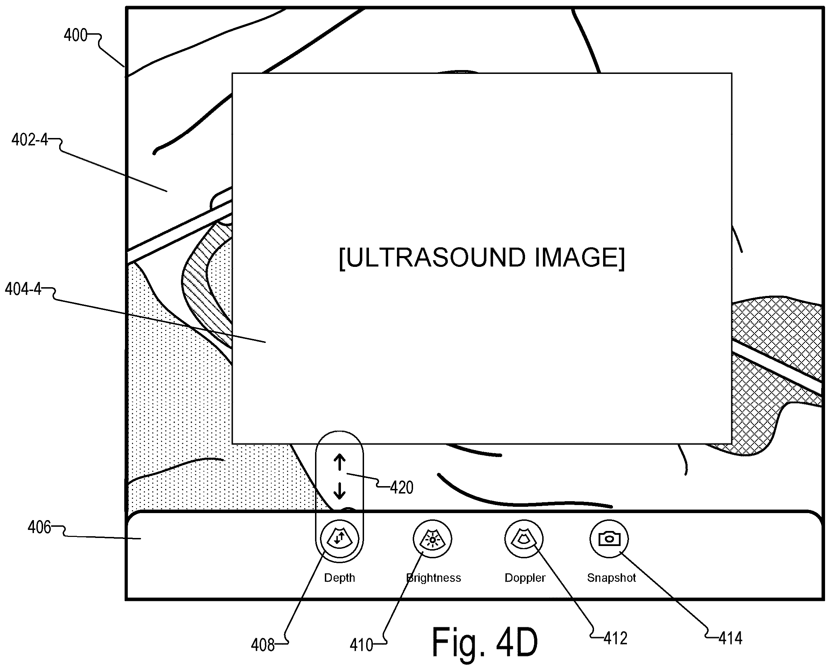

FIG. 4D illustrates an alternative example of interface 400 for the user to adjust the depth of an ultrasound image 404-4 in the hybrid input mode. Rather than depth slider 418, FIG. 4D shows depth arrows 420, by which the user may select deeper or shallower depths of focus of ultrasound probe 306. The interaction with depth arrows 420 using the second control may be similar to those of other icons and other parameter setting elements. As the user selects the up arrow of depth arrows 420 using the second control, ultrasound image 404-4 may show an ultrasound image from an increasingly shallower depth of anatomical object 302. As the user selects the down arrow of depth arrows 420 using the second control, ultrasound image 404-4 may show an ultrasound image from an increasingly deeper depth of anatomical object 302. In this manner, the hybrid input mode may allow for efficient viewing of different portions of anatomical object 302, allowing the user to concurrently adjust both an ultrasound scan location on anatomical object 302 and a depth of focus within anatomical object 302.

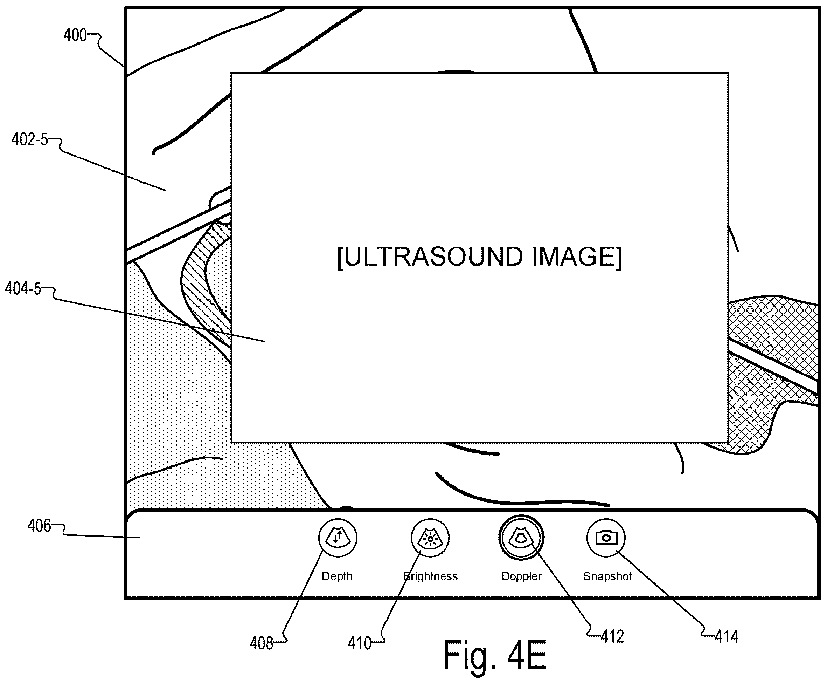

As another example, FIG. 4E illustrates the user selecting a doppler mode for ultrasound imagery in interface 400 in the hybrid input mode. As shown, the user has selected doppler icon 412, which may be indicated by a highlight of doppler icon 412, an outline of doppler icon 412, a change in color of doppler icon 412, or any other suitable indicator. The selection of doppler icon 412 may be in a manner as described with other icons (e.g., depth icon 408 and brightness icon 410). Based on the selection of doppler icon 412, an ultrasound image 404-5 may be an ultrasound image showing blood flow direction and/or any other information that may be captured by a doppler mode of ultrasound imaging.

While user interface element 406 shows four parameter settings that may be adjusted, more parameters, fewer parameters, or different parameters may be provided for adjustment in the hybrid input mode. For instance, other modes (e.g., modalities) of ultrasound may be adjusted in addition to or instead of doppler mode. Further, any other suitable parameter settings associated with ultrasound imaging may be provided for adjustment, such as a gain, a frequency, a width of image, a processing of imagery, a color, etc.

In certain examples, the hybrid input mode may allow the user to adjust an overall brightness of interface 400, a brightness of a portion or portions of interface 400 (e.g., images 402, images 402 and ultrasound images 404, etc.), a size, position, and/or characteristic (e.g., transparency, color, visibility, etc.) of ultrasound images 404 shown in interface 400, etc. For instance, the user may desire to see more of image 402, e.g., to see more clearly a location of ultrasound probe 306 while in the hybrid input mode. The hybrid input mode may provide as an adjustable parameter setting the size, position, transparency, visibility, or any other suitable characteristic of ultrasound images 404 so that ultrasound images 404 take up less space on interface 400 and/or otherwise allow image 402 or elements of interest depicted in image 402 to be visible.

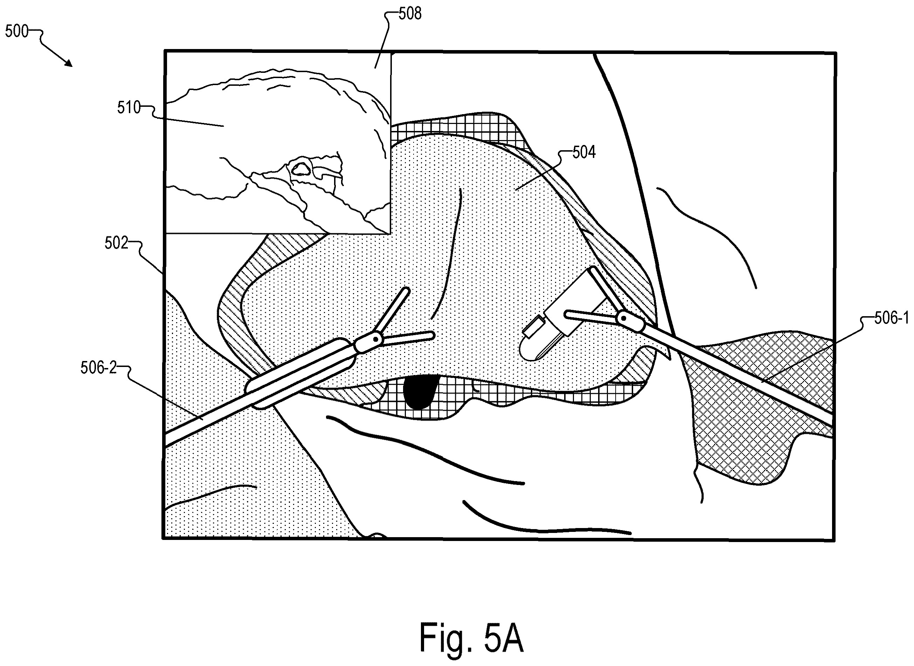

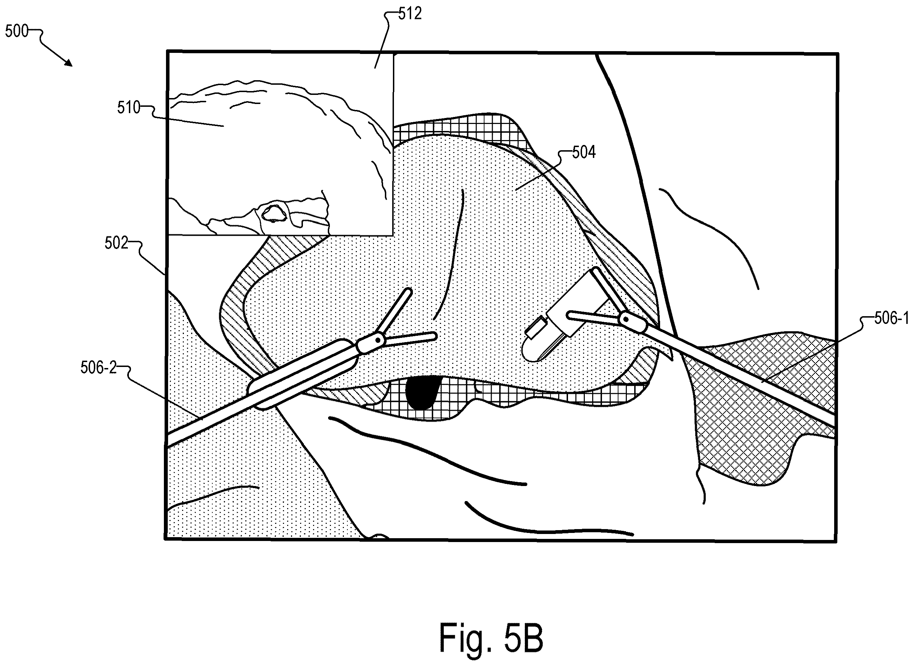

FIGS. 5A and 5B illustrate another example interface 500 for input modes for a computer-assisted medical system. Interface 500 shows an illustrative image 502 provided by the computer-assisted medical system. Image 502 may be an image of the anatomical space provided by an imaging device. Image 502 shows an anatomical object 504, which may be an organ on which the user is performing a medical procedure. Image 502 also shows a first instrument 506-1 and a second instrument 506-2. As described, first instrument 506-1 may be coupled to a first manipulator of the computer-assisted medical system and second instrument 506-2 may be coupled to a second manipulator of the computer-assisted medical system. Interface 500 further shows an inset image 508 (e.g., in a picture-in-picture window) that shows a 3D model 510, such as a 3D model of anatomical object 504 (e.g., a preoperative or intraoperative 3D model of anatomical object 504).

In a manipulation mode, the user may provide input to a first control of the set of controls to manipulate the first manipulator to cause first instrument 506-1 to move in the anatomical space. Similarly, the user may provide input to a second control of the set of controls to manipulate the second manipulator to cause second instrument 506-2 to move in the anatomical space. The user may further provide input to control the imaging device to change a view of image 502. For instance, the user may move the user's head, which may result in a corresponding movement of imaging device to show a corresponding change in angle or view of image 502. Additionally or alternatively, the imaging device may change the view of image 502 to follow a movement of first instrument 506-1 and/or second instrument 506-2. Any other suitable input may be provided to change image 502.

In the manipulation mode, in response to a change in image 502 and/or imaging device, image 508 may show a corresponding change to a view of 3D model 510. For instance, if the user causes the imaging device to zoom in to show a zoomed view of image 502, image 508 may show a similarly zoomed view of 3D model 510. If the user causes the imaging device to show a portion to the right of what is currently shown in image 502, image 508 may likewise show a portion of 3D model 510 to the right of what is currently shown in image 508. Thus, in the manipulation mode, image 508 may track on 3D model 510 based on what is shown of anatomical object 504 in image 502.

When system 100 configures the computer-assisted medical system to operate in a hybrid input mode, the user may continue to manipulate first instrument 506-1 by providing input to the first control to manipulate the first manipulator. Input provided by the user to the second control may be translated to adjust a display parameter setting associated with the medical procedure.

For instance, FIG. 5B illustrates interface 500 in the hybrid input mode. FIG. 5B shows interface 500 with image 502 showing anatomical object 504, first instrument 506-1, and second instrument 508-2. FIG. 5B further shows an inset image 512 that shows a different view of 3D model 510 (e.g., zoomed in to an upper portion of 3D model 510). In the hybrid input mode, the user may adjust the display parameter setting of inset image 512 to adjust a view of 3D model 510 by providing input to the second control. Thus, the user may zoom, rotate, translate, or otherwise adjust 3D model 510 and/or a camera view of 3D model 510 using inputs provided to the second control. Meanwhile, input provided to the first control may continue to manipulate the first manipulator to control first instrument 506-1. In some examples, as system 100 exits the hybrid input mode and returns to the manipulation input mode, image 512 may return back to an image that tracks image 502 (e.g., image 508). In other examples, image 512 may remain as the user manipulated and/or display a combination of a manipulated image and a tracking image (e.g., remain zoomed in but track a motion and/or angle of image 502).

In certain examples, the parameters provided for adjustment may be dependent on context of the medical procedure. For example, the parameters provided for adjustment may be based on the instrument being controlled by the first control in the hybrid input mode. For instance, when an ultrasound probe is being controlled by user input to the first control in the hybrid input mode, parameters related to ultrasound and/or the ultrasound probe may be provided for adjustment by the second control. If a different instrument (e.g., a suturing tool) were being manipulated by the first control, different parameters related to the different instrument (e.g., a type of suture, a technique of suturing, etc.) may be presented for adjustment.

In some examples, system 100 may be configured to provide various mechanisms to assist a user of a computer-assisted medical system with operation in hybrid input mode. Such mechanisms may include mechanisms configured to mitigate risks and/or assist the user in handling an increased or different cognitive load than that associated with use of a manipulation input mode. For example, user input system 100 may include mechanisms to make abundantly clear when the user enters and exits the hybrid input mode. For instance, user input system 100 may be configured to provide a notification such as an alert upon entering and/or exiting the hybrid input mode. The notification may include one or more notifications and/or types of notifications, such as an audio notification, a visual notification, a haptic notification, etc.

In certain examples, user input system 100 may require relatively elaborate and/or deliberate input to enter and/or exit the hybrid input mode. For example, the input may include a series of inputs in a specified order, an input from an input mechanism that is not easily triggered, a plurality of inputs in succession on an input mechanism (e.g., a double-click, triple-click, etc.), a duration of time of input (e.g., holding a button for a predetermined amount of time, etc.), or any other suitable input that may mitigate an unintentional entering and/or exiting of the hybrid input mode.

In certain examples, user input system 100 may include mechanisms to make abundantly clear to the user that user input system 100 is operating in the hybrid input mode. For example, user input system 100 may provide a visualization of a user interface and/or images presented to the user in the hybrid input mode that is different from a visualization of a user interface and/or images presented to the user in the manipulation input mode and/or another mode of operation. Such different visualizations may include any suitable visual mechanisms, such as an addition of a prominent parameter setting user interface element, a changing of a visualization parameter (e.g., changing a color, changing to grayscale, changing a style of visualization, inverting the images, changing a size of the images, etc.), etc. Additionally or alternatively, such mechanisms may include an audio indicator indicating that the user is in the hybrid mode, such as a periodic beep or other alert, a playback of a sound for a duration of the hybrid mode, or any other suitable audio indicator.

In certain examples, user input system 100 may include mechanisms that transition the user into or out of the hybrid input mode. For example, user input system 100 may filter inputs received on controls for a predetermined amount of time upon entering and/or exiting the hybrid input mode. For instance, user input system 100 may filter out movements of the controls that exceed a threshold velocity and/or distance for an amount of time (e.g., a second, a portion of a second, several seconds, etc.) upon entering and/or exiting the hybrid input mode. Additionally or alternatively, user input system 100 may filter all movement (e.g., prevent all movement of controls and/or instruments) for a predetermined amount of time upon entering and/or exiting the hybrid input mode. Additionally or alternatively, user input system 100 may temporarily constrain movement of the controls, manipulators, and/or instruments, for instance, by setting a velocity of the controls, manipulators, and/or instrument upon entering and/or exiting the hybrid input mode. For example, user input system 100 may initially set the velocity of the controls, manipulators, and/or instruments to zero for a predetermined amount of time upon entering and/or exiting the hybrid input mode. User input system 100 may constrain movement of the controls, manipulators, and/or instruments in any other suitable manner.

In certain examples, user input system 100 may include mechanisms that restrict operation of both controls simultaneously in the hybrid input mode. For example, user input system 100 may filter inputs received on controls so that when user input is received on one control, user input system 100 may refrain from translating user input received on the other control. In this manner, user input system 100 may allow a user to either manipulate a manipulator or adjust a parameter setting one at a time but not simultaneously. User input system 100 may implement such a restriction in any suitable manner.

User input system 100 may include any of the above-described example mechanisms, any combination or sub-combination thereof, and/or any other suitable mechanisms to clearly demarcate operation in the hybrid input mode so that the user is made fully aware of the effects of inputs provided at the controls.

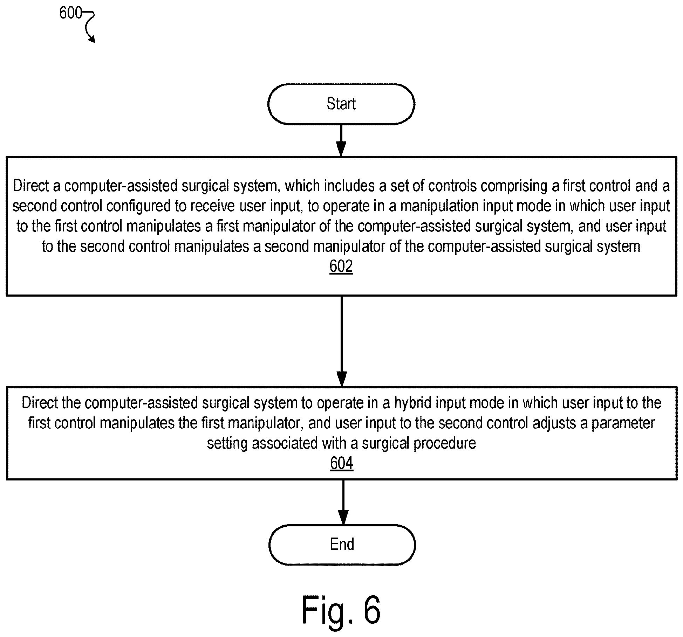

FIG. 6 illustrates an example user input method 600 for a computer-assisted medical system. While FIG. 6 illustrates example operations according to one embodiment, other embodiments may omit, add to, reorder, combine, and/or modify any of the operations shown in FIG. 6. One or more of the operations shown in in FIG. 6 may be performed by a user input system such as user input system 100, any components included therein, and/or any implementation thereof.

In operation 602, a user input system may direct a computer-assisted medical system, which includes a set of controls comprising a first control and a second control configured to receive user input, to operate in a manipulation input mode in which user input to the first control manipulates a first manipulator of the computer-assisted medical system, and user input to the second control manipulates a second manipulator of the computer-assisted medical system. Operation 602 may be performed in any of the ways described herein.

In operation 604, the user input system may direct the computer-assisted medical system to operate in a hybrid input mode in which user input to the first control manipulates the first manipulator, and user input to the second control adjusts a parameter setting associated with a medical procedure. Operation 604 may be performed in any of the ways described herein.

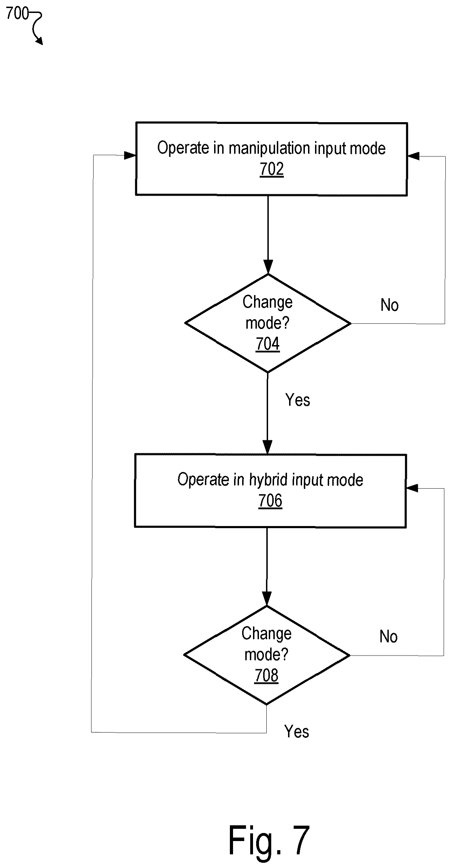

FIG. 7 illustrates an example user input method 700 for a computer-assisted medical system. While FIG. 7 illustrates example operations according to one embodiment, other embodiments may omit, add to, reorder, combine, and/or modify any of the operations shown in FIG. 7. One or more of the operations shown in in FIG. 7 may be performed by a user input system such as user input system 100, any components included therein, and/or any implementation thereof.

In operation 702, the user input system operates in a manipulation input mode. For example, the user input system may configure the computer-assisted medical system to operate based on the manipulation input mode. Operation 702 may be performed in any of the ways described herein.

In operation 704, the user input system determines whether to change to operation in another input mode. The determination may be based on user input received by the user input system, and may include the user input system determining whether received user input matches a predefined command to change modes. If no, the user input system may return to operation 702 and continue to operate in the manipulation input mode. If yes, the user input system may move to operation 706 to change the active input mode from the manipulation input mode to the hybrid input mode.

In operation 706, the user input system operates in a hybrid input mode. For example, the user input system may configure the computer-assisted medical system to operate based on the hybrid input mode. Operation 706 may be performed in any of the ways described herein.

In operation 708, the user input system determines whether to change to operation in another input mode. Similar to operation 704, the determination may be based on user input received by the user input system, and may include the user input system determining whether received user input matches a predefined command to change modes. If no, the user input system my return to operation 706 and continue to operate in the hybrid input mode. If yes, the user input system may move to operation 702 to change the active input mode from the hybrid input mode to the manipulation input mode.

Any suitable user input may be defined and used to trigger a transition from the manipulation input mode to the hybrid input mode and/or a transition from the hybrid input mode to the manipulation input mode. The same user input or different user input may be used to trigger each transition.

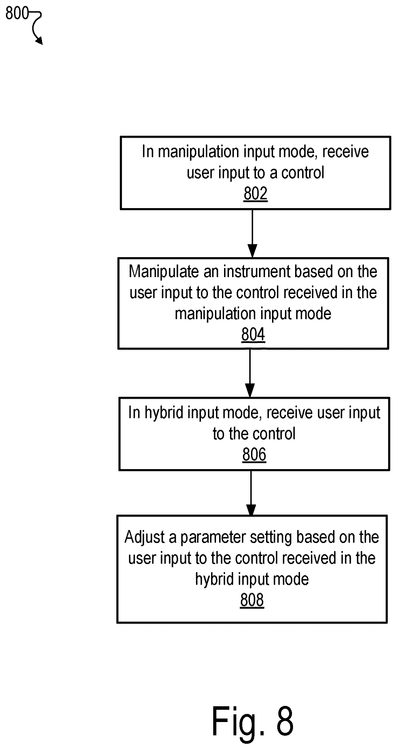

FIG. 8 illustrates an example user input method 800 for a computer-assisted medical system. While FIG. 8 illustrates example operations according to one embodiment, other embodiments may omit, add to, reorder, combine, and/or modify any of the operations shown in FIG. 8. One or more of the operations shown in in FIG. 8 may be performed by a user input system such as user input system 100, any components included therein, and/or any implementation thereof.

In operation 802, the user input system, while operating in a manipulation input mode, receives user input to a control. Operation 802 may be performed in any of the ways described herein.

In operation 804, the user input system manipulates an instrument based on the user input to the control received in the manipulation input mode. Operation 804 may be performed in any of the ways described herein.

In operation 806, the user input system, while operating in a hybrid input mode, receives user input to the control. In some examples, the input received may include a same type of input (e.g., a manipulation, an actuation of a button, etc.) as received in the manipulation input mode in operation 802. Operation 806 may be performed in any of the ways described herein.

In operation 808, the user input system adjusts a parameter setting based on the user input to the control received in the hybrid input mode. Operation 808 may be performed in any of the ways described herein.

FIG. 9 shows an illustrative computer-assisted medical system 900 ("medical system 900"). System 100 may be implemented by medical system 900, connected to medical system 900, and/or otherwise used in conjunction with medical system 900.

As shown, medical system 900 may include a manipulating system 902, a user control system 904, and an auxiliary system 906 communicatively coupled one to another. Medical system 900 may be utilized by a medical team to perform a computer-assisted medical procedure on a patient 908. As shown, the medical team may include a surgeon 910-1, an assistant 910-2, a nurse 910-3, and an anesthesiologist 910-4, all of whom may be collectively referred to as "medical team members 910." Additional or alternative medical team members may be present during a medical session as may serve a particular implementation.

While FIG. 9 illustrates an ongoing minimally invasive procedure, it will be understood that medical system 900 may similarly be used to perform open surgical procedures or other types of medical procedures that may similarly benefit from the accuracy and convenience of medical system 900. Additionally, it will be understood that the medical session throughout which medical system 900 may be employed may not only include an operative phase of a medical procedure, as is illustrated in FIG. 9, but may also include preoperative, postoperative, and/or other suitable phases of the medical procedure.

As shown in FIG. 9, manipulating system 902 may include a plurality of manipulator arms 912 (e.g., manipulator arms 912-1 through 912-4) to which a plurality of medical instruments may be coupled. Each medical instrument may be implemented by any suitable therapeutic instrument (e.g., a tool having tissue-interaction functions), medical tool, imaging device (e.g., an endoscope, an ultrasound probe, etc.), diagnostic instrument, or the like that may be used for a computer-assisted medical procedure on patient 908 (e.g., by being at least partially inserted into patient 908 and manipulated to perform a computer-assisted medical procedure on patient 908). In some examples, one or more of the medical instruments may include force-sensing and/or other sensing capabilities. While manipulating system 902 is depicted and described herein as including four manipulator arms 912, it will be recognized that manipulating system 902 may include only a single manipulator arm 912 or any other number of manipulator arms as may serve a particular implementation.

Manipulator arms 912 and/or medical instruments attached to manipulator arms 912 may include one or more displacement transducers, orientational sensors, and/or positional sensors used to generate raw (i.e., uncorrected) kinematics information. One or more components of medical system 900 may be configured to use the kinematics information to track (e.g., determine positions of) and/or control the medical instruments.

User control system 904 may be configured to facilitate control by surgeon 910-1 of manipulator arms 912 and medical instruments attached to manipulator arms 912. For example, surgeon 910-1 may interact with user control system 904 to remotely manipulate manipulator arms 912 and the medical instruments. To this end, user control system 904 may provide surgeon 910-1 with imagery (e.g., high-definition 3D imagery) of a surgical area associated with patient 908 as captured by an imaging system (e.g., any of the medical imaging systems described herein). In certain examples, user control system 904 may include a stereo viewer having two displays where stereoscopic images of an anatomical space associated with patient 908 and generated by a stereoscopic imaging system may be viewed by surgeon 910-1. Surgeon 910-1 may utilize the imagery to perform one or more procedures with one or more medical instruments attached to manipulator arms 912.

To facilitate control of medical instruments, user control system 904 may include a set of master controls. These master controls may be manipulated by surgeon 910-1 to control movement of medical instruments (e.g., by utilizing robotic and/or teleoperation technology). The master controls may be configured to detect a wide variety of hand, wrist, and finger movements by surgeon 910-1. User control system 904 may be configured to receive from the master controls information such as position, movement, rotation, interaction with etc., to track movement of the master controls. User control system 904 may translate the received information into corresponding movement and/or control of manipulator arms 912. In this way, surgeon 910-1 may manipulate medical instruments attached to manipulator arms 912 using the master controls. In this manner, surgeon 910-1 may intuitively perform a procedure using one or more medical instruments.

Auxiliary system 906 may include one or more computing devices configured to perform primary processing operations of medical system 900. In such configurations, the one or more computing devices included in auxiliary system 906 may control and/or coordinate operations performed by various other components (e.g., manipulating system 902 and user control system 904) of medical system 900. For example, a computing device included in user control system 904 may transmit instructions to manipulating system 902 by way of the one or more computing devices included in auxiliary system 906. As another example, auxiliary system 906 may receive, from manipulating system 902, and process image data representative of imagery captured by an imaging device attached to one of manipulator arms 912.

In some examples, auxiliary system 906 may be configured to present visual content to medical team members 910 who may not have access to the images provided to surgeon 910-1 at user control system 904. To this end, auxiliary system 906 may include a display monitor 914 configured to display one or more user interfaces, such as images (e.g., 2D images, 3D images) of the surgical area, information associated with patient 908 and/or the medical procedure, and/or any other visual content as may serve a particular implementation. For example, display monitor 914 may display images of the surgical area together with additional content (e.g., graphical content, contextual information, etc.) concurrently displayed with the images. In some embodiments, display monitor 914 is implemented by a touchscreen display with which medical team members 910 may interact (e.g., by way of touch gestures) to provide user input to medical system 900.

Manipulating system 902, user control system 904, and auxiliary system 906 may be communicatively coupled one to another in any suitable manner. For example, as shown in FIG. 9, manipulating system 902, user control system 904, and auxiliary system 906 may be communicatively coupled by way of control lines 916, which may represent any wired or wireless communication link as may serve a particular implementation. To this end, manipulating system 902, user control system 904, and auxiliary system 906 may each include one or more wired or wireless communication interfaces, such as one or more local area network interfaces, Wi-Fi network interfaces, cellular interfaces, etc.

Certain examples described herein are directed to implementations of system 100 with computer-assisted medical systems such as medical system 900. In such implementations, system 100 may be configured to selectively direct medical system 900 to operate in a manipulation input mode or a hybrid input mode as described herein. The actively operating input mode governs how user input received by master controls is translated to operations of medical system 900. In other examples, system 100 may be similarly implemented with other computer-assisted systems (e.g., surgical systems), robotic systems, etc.

In some examples, a non-transitory computer-readable medium storing computer-readable instructions may be provided in accordance with the principles described herein. The instructions, when executed by a processor of a computing device, may direct the processor and/or computing device to perform one or more operations, including one or more of the operations described herein. Such instructions may be stored and/or transmitted using any of a variety of known computer-readable media.

A non-transitory computer-readable medium as referred to herein may include any non-transitory storage medium that participates in providing data (e.g., instructions) that may be read and/or executed by a computing device (e.g., by a processor of a computing device). For example, a non-transitory computer-readable medium may include, but is not limited to, any combination of non-volatile storage media and/or volatile storage media. Illustrative non-volatile storage media include, but are not limited to, read-only memory, flash memory, a solid-state drive, a magnetic storage device (e.g. a hard disk, a floppy disk, magnetic tape, etc.), ferroelectric random-access memory ("RAM"), and an optical disc (e.g., a compact disc, a digital video disc, a Blu-ray disc, etc.). Illustrative volatile storage media include, but are not limited to, RAM (e.g., dynamic RAM).

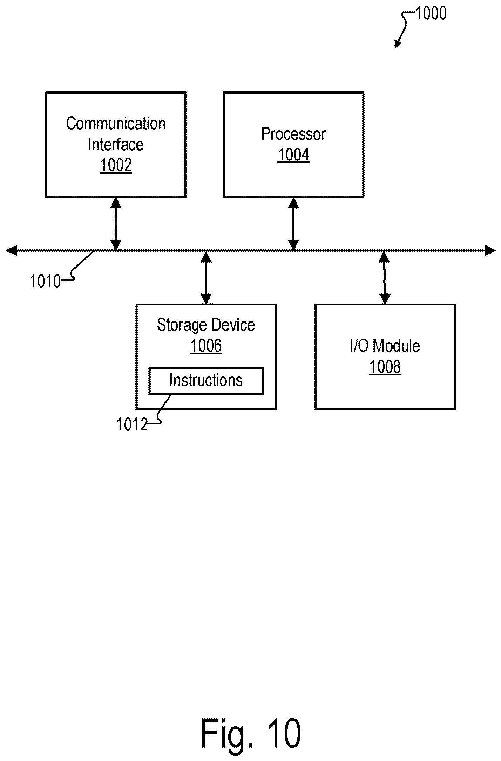

FIG. 10 illustrates an example computing device 1000 that may be specifically configured to perform one or more of the processes described herein. Any of the systems, units, computing devices, and/or other components described herein may be implemented by computing device 1000.

As shown in FIG. 10, computing device 1000 may include a communication interface 1002, a processor 1004, a storage device 1006, and an input/output ("I/O") module 1008 communicatively connected one to another via a communication infrastructure 1010. While an example computing device 1000 is shown in FIG. 10, the components illustrated in FIG. 10 are not intended to be limiting. Additional or alternative components may be used in other embodiments. Components of computing device 1000 shown in FIG. 10 will now be described in additional detail.

Communication interface 1002 may be configured to communicate with one or more computing devices. Examples of communication interface 1002 include, without limitation, a wired network interface (such as a network interface card), a wireless network interface (such as a wireless network interface card), a modem, an audio/video connection, and any other suitable interface.

Processor 1004 generally represents any type or form of processing unit capable of processing data and/or interpreting, executing, and/or directing execution of one or more of the instructions, processes, and/or operations described herein. Processor 1004 may perform operations by executing computer-executable instructions 1012 (e.g., an application, software, code, and/or other executable data instance) stored in storage device 1006.

Storage device 1006 may include one or more data storage media, devices, or configurations and may employ any type, form, and combination of data storage media and/or device. For example, storage device 1006 may include, but is not limited to, any combination of the non-volatile media and/or volatile media described herein. Electronic data, including data described herein, may be temporarily and/or permanently stored in storage device 1006. For example, data representative of computer-executable instructions 1012 configured to direct processor 1004 to perform any of the operations described herein may be stored within storage device 1006. In some examples, data may be arranged in one or more databases residing within storage device 1006.

I/O module 1008 may include one or more I/O modules configured to receive user input and provide user output. I/O module 1008 may include any hardware, firmware, software, or combination thereof supportive of input and output capabilities. For example, I/O module 1008 may include hardware and/or software for capturing user input, including, but not limited to, a keyboard or keypad, a touchscreen component (e.g., touchscreen display), a receiver (e.g., an RF or infrared receiver), motion sensors, and/or one or more input buttons.

I/O module 1008 may include one or more devices for presenting output to a user, including, but not limited to, a graphics engine, a display (e.g., a display screen), one or more output drivers (e.g., display drivers), one or more audio speakers, and one or more audio drivers. In certain embodiments, I/O module 1008 is configured to provide graphical data to a display for presentation to a user. The graphical data may be representative of one or more graphical user interfaces and/or any other graphical content as may serve a particular implementation.

In some examples, any of the facilities described herein may be implemented by or within one or more components of computing device 1000. For example, one or more applications 1012 residing within storage device 1006 may be configured to direct an implementation of processor 1004 to perform one or more operations or functions associated with processing facility 104 of system 100. Likewise, storage facility 102 of system 100 may be implemented by or within an implementation of storage device 1006.

One or more operations described herein may be performed in real time. As used herein, operations that are performed "in real time" will be understood to be performed immediately and without undue delay, even if it is not possible for there to be absolutely zero delay.

Any of the systems, devices, and/or components thereof may be implemented in any suitable combination or sub-combination. For example, any of the systems, devices, and/or components thereof may be implemented as an apparatus configured to perform one or more of the operations described herein.

In the preceding description, various illustrative embodiments have been described with reference to the accompanying drawings. It will, however, be evident that various modifications and changes may be made thereto, and additional embodiments may be implemented, without departing from the scope of the invention as set forth in the claims that follow. For example, certain features of one embodiment described herein may be combined with or substituted for features of another embodiment described herein. The description and drawings are accordingly to be regarded in an illustrative rather than a restrictive sense.
The present application also includes the following numbered clauses:
1. A system comprising:
   a memory storing instructions;
   a processor communicatively coupled to the memory and configured to execute the instructions to:
      direct a computer-assisted medical system, which includes a set of controls comprising a first control and a second control configured to receive user input, to operate in a manipulation input mode in which
         user input to the first control manipulates a first manipulator of the computer-assisted medical system, and
         user input to the second control manipulates a second manipulator of the computer-assisted medical system; and
      direct the computer-assisted medical system to operate in a hybrid input mode in which
         user input to the first control manipulates the first manipulator, and
         user input to the second control adjusts a parameter setting associated with a medical procedure.
2. The system of clause 1, wherein the user input to the second control that adjusts the parameter setting in the hybrid input mode comprises a same type of input as the user input to the second control that manipulates the second manipulator in the manipulation input mode.
3. The system of clause 1, wherein the parameter setting associated with the medical procedure includes a display parameter setting associated with the medical procedure.
4. The system of clause 3, wherein the display parameter setting associated with the medical procedure includes a display parameter of a three-dimensional (3D) model associated with the medical procedure.
5. The system of clause 1, wherein the parameter setting associated with the medical procedure includes an instrument parameter setting associated with an instrument controlled by the first manipulator.
6. The system of clause 1, wherein:
   the first manipulator is configured to manipulate an ultrasound probe; and
   the parameter setting includes at least one of:
      a gain of an ultrasound image from the ultrasound probe,
      a depth of the ultrasound image, or
      a modality of the ultrasound probe.
7. The system of clause 1, wherein the processor is further configured to execute the instructions to direct the computer-assisted medical system to provide a notification upon at least one of entering or exiting the hybrid input mode.
8. The system of clause 1, wherein the processor is further configured to execute the instructions to:
   receive a command to exit the hybrid input mode;
   direct, based on the command, the computer-assisted medical system to exit the hybrid input mode; and
   direct, upon exiting the hybrid input mode, the computer-assisted medical system to temporarily constrain movement of the manipulators.
9. The system of clause 1, wherein the user input to the first control that manipulates the first manipulator in the hybrid input mode and the user input to the second control that adjusts the parameter setting in the hybrid input mode are received concurrently.
10. A method comprising:
   directing, by a processor, a computer-assisted medical system, which includes a set of controls comprising a first control and a second control configured to receive user input, to operate in a manipulation input mode in which
      user input to the first control manipulates a first manipulator of the computer-assisted medical system, and
      user input to the second control manipulates a second manipulator of the computer-assisted medical system; and
   directing, by the processor, the computer-assisted medical system to operate in a hybrid input mode in which
      user input to the first control manipulates the first manipulator, and
      user input to the second control adjusts a parameter setting associated with a medical procedure.
11. The method of clause 10, wherein the user input to the second control that adjusts the parameter setting in the hybrid input mode comprises a same type of input as the user input to the second control that manipulates the second manipulator in the manipulation input mode.
12. The method of clause 10, wherein the parameter setting associated with the medical procedure includes a display parameter setting associated with the medical procedure.
13. The method of clause 10, wherein the parameter setting associated with the medical procedure includes an instrument parameter setting associated with an instrument controlled by the first manipulator.
14. The method of clause 10, wherein:
   the first manipulator is configured to manipulate an ultrasound probe; and
   the parameter setting includes at least one of:
      a gain of an ultrasound image from the ultrasound probe,
      a depth of the ultrasound image, or
      a modality of the ultrasound probe.
15. The method of clause 10, further comprising directing, by the processor, the computer-assisted medical system to provide a notification upon at least one of entering or exiting the hybrid input mode.
16. The method of clause 10, further comprising:
   receiving, by the processor, a command to exit the hybrid input mode;
   directing, by the processor and based on the command, the computer-assisted medical system to exit the hybrid input mode; and
   directing, by the processor and upon exiting the hybrid input mode, the computer-assisted medical system to temporarily constrain movement of the manipulators.
17. The method of clause 10, wherein the user input to the first control that manipulates the first manipulator in the hybrid input mode and the user input to the second control that adjusts the parameter setting in the hybrid input mode are received concurrently.
18. A non-transitory computer-readable medium storing instructions that, when executed by a processor, cause the processor to:
   direct a computer-assisted medical system, which includes a set of controls comprising a first control and a second control configured to receive user input, to operate in a manipulation input mode in which
      user input to the first control manipulates a first manipulator of the computer-assisted medical system, and
      user input to the second control manipulates a second manipulator of the computer-assisted medical system; and
   direct the computer-assisted medical system to operate in a hybrid input mode in which
      user input to the first control manipulates the first manipulator, and
      user input to the second control adjusts a parameter setting associated with a medical procedure.
19. The non-transitory computer-readable medium of clause 18, wherein the user input that adjusts the parameter setting in the hybrid input mode comprises a same type of input as the user input to the second control that manipulates the second manipulator in the manipulation input mode.
20. The non-transitory computer-readable medium of clause 18, wherein the parameter setting associated with the medical procedure includes a display parameter setting associated with the medical procedure.
21. The non-transitory computer-readable medium of clause 18, wherein the parameter setting associated with the medical procedure includes an instrument parameter setting associated with an instrument controlled by the first manipulator.
22. The non-transitory computer-readable medium of clause 18, wherein: the first manipulator is configured to manipulate an ultrasound probe; and the parameter setting includes at least one of:
   a gain of an ultrasound image from the ultrasound probe,
   a depth of the ultrasound image, or
   a modality of the ultrasound probe.
23. The non-transitory computer-readable medium of clause 18, wherein the instructions, when executed, further cause the processor to direct the computer-assisted medical system to provide a notification upon at least one of entering or exiting the hybrid input mode.
24. The non-transitory computer-readable medium of clause 18, wherein the instructions, when executed, further cause the processor to:
   receive a command to exit the hybrid input mode;
   direct, based on the command, the computer-assisted medical system to exit the hybrid input mode; and
   direct, upon exiting the hybrid input mode, the computer-assisted medical system to temporarily constrain movement of the manipulators.
25. The non-transitory computer-readable medium of clause 18, wherein the user input to the first control that manipulates the first manipulator in the hybrid input mode and the user input to the second control that adjusts the parameter setting in the hybrid input mode are received concurrently.

## Claims

1. A system (100) comprising:
a memory (102) storing instructions (106);
a processor (104) communicatively coupled to the memory (102) and configured to execute the instructions (106) to:
direct a computer-assisted medical system (202), which includes a set of controls comprising a first control (204-1) and a second control (204-2) configured to receive user input, to operate in a hybrid input mode (208-2) comprising:
receiving first user input to a first control (204-1) configured to manipulate a first manipulator (206-1) of the computer-assisted medical system (202);
translating the first user input into manipulation of the first manipulator (206-1);
receiving second user input to a second control (204-2) configured, in a manipulation input mode (208-1), to manipulate a second manipulator (206-2) of the computer-assisted medical system (202); and
adjusting, based on the second user input to the second control (204-2), a parameter setting associated with a medical procedure.

2. The system (100) of claim 1, wherein the second user input to the second control (204-2) that adjusts the parameter setting in the hybrid input mode (208-2) comprises a same type of input as a user input to the second control (204-2) that manipulates the second manipulator (206-2) in the manipulation input mode (208-1).

3. The system (100) of claim 1, wherein the parameter setting associated with the medical procedure includes a display parameter setting associated with the medical procedure.

4. The system (100) of claim 3, wherein the display parameter setting associated with the medical procedure includes a display parameter of a three-dimensional, 3D, model associated with the medical procedure.

5. The system (100) of claim 1, wherein the parameter setting associated with the medical procedure includes an instrument parameter setting associated with an instrument controlled by the first manipulator (206-1).

6. The system (100) of claim 1, wherein the processor (104) is further configured to execute the instructions (106) to direct the computer-assisted medical system (202) to provide a notification upon at least one of entering or exiting the hybrid input mode (208-2).

7. The system (100) of claim 1, wherein the processor (104) is further configured to execute the instructions (106) to:
receive a command to exit the hybrid input mode (208-2);
direct, based on the command, the computer-assisted medical system (202) to exit the hybrid input mode (208-2); and
direct, upon exiting the hybrid input mode (208-2), the computer-assisted medical system (202) to temporarily constrain movement of the manipulators.

8. The system (100) of claim 1, wherein the first user input to the first control (204-1) that manipulates the first manipulator (206-1) in the hybrid input mode (208-2) and the second user input to the second control (204-2) that adjusts the parameter setting in the hybrid input mode (208-2) are received concurrently.

9. The system (100) of claim 1, wherein:
the first manipulator (206-1) is configured to manipulate an ultrasound probe (306); and
the parameter setting includes a gain of an ultrasound image from the ultrasound probe (306).

10. The system (100) of claim 1, wherein:
the first manipulator (206-1) is configured to manipulate an ultrasound probe (306); and
the parameter setting includes a depth of the ultrasound image.

11. The system (100) of claim 1, wherein:
the first manipulator (206-1) is configured to manipulate an ultrasound probe (306); and
the parameter setting includes a modality of the ultrasound probe (306).

12. The system (100) of claim 1, wherein:
the first manipulator (206-1) is configured to manipulate a cauterizing instrument; and
the parameter setting includes a power output of the cauterizing instrument.

13. The system (100) of claim 1, wherein the parameter setting includes an imaging parameter setting of an imaging device associated with the medical procedure.

14. The system (100) of claim 1, wherein:
the processor (104) is further configured to execute the instructions (106) to direct the computer-assisted medical system (202) to operate in the manipulation input mode (208-1), wherein user input to the second control (204-2) is translated into a movement of the second manipulator (206-2) of the computer-assisted medical system (202); and
when the processor (104) directs the computer-assisted medical system (202) to operate in the hybrid input mode (208-2), a same type of user input to the second control (204-2) is translated to an adjustment of the parameter setting associated with the medical procedure.

15. A method comprising:
directing, by a processor (104), a user input system to operate in a hybrid input mode (208-2) comprising:
receiving first user input to a first control (204-1) configured to manipulate a first robotic manipulator;
translating the first user input into movement of the first robotic manipulator (206-1) to teleoperate a first instrument (304-1);
receiving second user input to a second control (204-2) configured, in a manipulation input mode (208-1), to manipulate a second robotic manipulator; and
translating the second user input to the second control (204-2) into an adjustment of a parameter setting of the first instrument (304-1).
